# EUROPEAN PATENT APPLICATION

(11) **EP 4 505 936 A1**
(43) Date of publication of application: **12.02.2025**
(21) Application number: 23811482.1
(22) Date of filing: 10.04.2023
(51) Int. Cl.: A47L 13/17, A47L 13/20, A01N 33/12, A01P 1/00, A61L 2/18, A47K 7/00, A01N 43/40, A01N 25/34, A01N 47/12, A01N 47/44

(54) **CLEANSING WET WIPE**

(30) Priority: 24.05.2022 JP 2022084195
(71) Applicant: Daio Paper Corporation, Shikokuchuo-shi, Ehime 799-0492 (JP)
(72) Inventor: MIURA, Akiteru, Shikokuchuo-shi, Ehime 799-0113 (JP)
(74) Representative: Bandpay & Greuter
(86) International application number: PCT/JP2023/014538
(87) International publication number: WO 2023/228604

(57) **Abstract**

A cleansing wet wipe 100 in which a base paper sheet is impregnated with a chemical liquid, wherein: the chemical liquid contains at least iodopropynyl butylcarbamate, polyamino-propyl biguanide, benzalkonium chloride, and cetylpyridinium chloride; and the content value for the iodopropynyl butylcarbamate in the chemical liquid is 0.005-0.02 mass% inclusive. The chemical liquid is adjusted to be acidic.

## Description

### TECHNICAL FIELD

The present invention relates to a cleansing wet wipe capable of suppressing mold caused on a cleaned surface after wiping.

### BACKGROUND ART

In the related art, there are wet wipes (wet tissues) that are used to wipe off contamination, sweat, and the like from bodies. Among such wet wipes, there are stacked-type sheets and roll-type sheets, and a plurality of wet wipes are accommodated in predetermined bags or containers.

Although there has been a demand for wet wipes that do not use ethanol as wet wipes that can be used safely by users who are sensitive to skin irritation such as babies and infants recently, there is a concern that if ethanol is not used, mold and the like may grow in the wet wipes inside the bags or containers before the plurality of sheets are used up.

Therefore, wet wipes (wet tissues) capable of preventing mold and the like from growing even with no use of ethanol have been proposed (see Patent Document 1, for example).

### CITATION LIST

### Patent Literature

Patent Document 1: JP 6795889B

### SUMMARY OF INVENTION

### Technical Problem

Incidentally, although anti-mold smoking agents are used to prevent mold from growing in bathrooms, the anti-mold smoking agents cannot remove mold that has already been caused.

Therefore, although wall surfaces, ceilings, and the like are typically cleaned by spraying chemical solutions in a case where mold that has already been caused is removed, the chemical solutions applied to the ceilings may drip, which requires attention and makes mold removal cleaning complicated.

Thus, the present inventors conducted intensive studies and developed a cleansing wipe capable of applying an anti-mold effect to a cleaned surface after wiping while wiping off contamination such as mold.

An object of the present invention is to provide a cleansing wet wipe capable of suppressing mold caused on a cleaned surface after wiping.

### Solution to Problem

In order to solve the above problem, the invention according to aspect 1 is
a cleansing wet wipe obtained by impregnating a base paper sheet with a chemical solution,
in which the chemical solution contains at least iodopropynyl butylcarbamate, polyaminopropyl biguanide, benzalkonium chloride, and cetylpyridinium chloride.

According to the invention of aspect 2, in the cleansing wet wipe according to aspect 1,
in the chemical solution, content of iodopropynyl butylcarbamate is equal to or greater than 0.005% by mass and equal to or less than 0.02% by mass.

According to the invention of aspect 3, in the cleansing wet wipe according to aspect 1,
the chemical solution is adjusted to show acidity.

According to the invention of aspect 4, in the cleansing wet wipe according to any one of aspects 1 to 3,
a plurality of projecting embossed parts which are embossed parts projecting from a first surface and a plurality of recessed embossed parts which are embossed parts projecting from a second surface are formed in the base paper sheet.

According to the invention of aspect 5, in the cleansing wet wipe according to aspect 4,
the cleansing wet wipe is a wet wipe for cleaning a bathroom.

### Advantageous Effects of Invention

According to the present invention, it is possible to obtain a cleansing wet wipe capable of suppressing mold caused on a cleaned surface after wiping.

### BRIEF DESCRIPTION OF DRAWINGS

[FIG. 1] This is a perspective view illustrating a cleaning tool to which a cleansing wet wipe according to the present embodiment is attached.
[FIG. 2] This is a plan view illustrating the cleansing wet wipe according to the present embodiment.

### DESCRIPTION OF EMBODIMENT

Hereinafter, a cleansing wet wipe 100 according to an embodiment of the present invention will be described with reference to the drawings. However, the technical scope of the present invention is not limited to the illustrated example and is determined merely on the basis of the description of the claims.

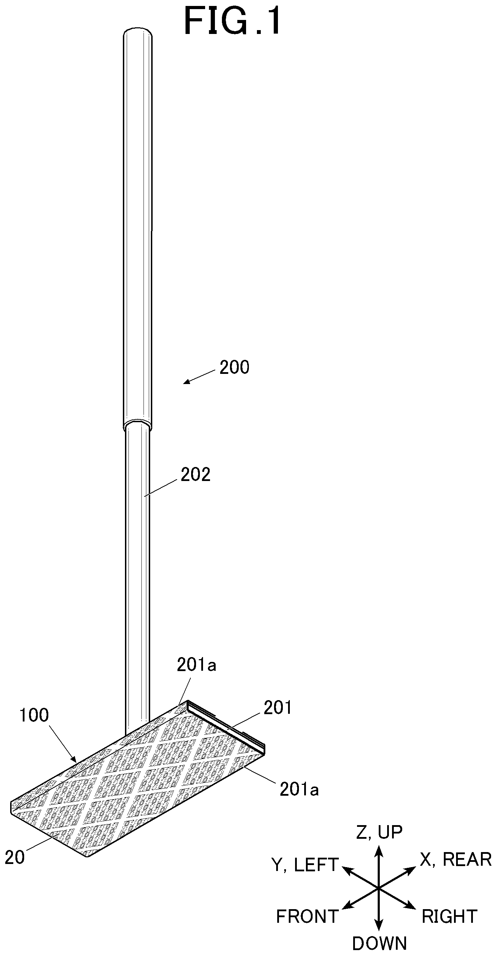

Note that the following description will be given by defining front-back, left-right, up-down, an X direction, a Y direction, and a Z direction as illustrated in FIG. 1. Also, "... to ..." in the specification will be used as a meaning of including numerical values described before and after "to" as a lower limit value and an upper limit value.

### (1. Description of configuration)

First, a configuration of the cleansing wet wipe 100 will be described.

### ((1) State during use)

As illustrated in FIG. 1, the cleansing wet wipe 100 is a sheet that is attached, in an exchangeable manner, to a cleaning tool 200 including a head portion 201 with a rectangular flat plate shape and a handle portion 202 that is attached to an upper surface of the head portion 201, for example, and is used to clean a floor surface, a wall surface, and a ceiling, and is a sheet obtained by impregnating a base paper sheet with a chemical solution.

Using the cleansing wet wipe 100 by attaching the cleansing wet wipe 100 to the head portion 201 of the cleaning tool 200 including the handle portion 202 with a long bar shape makes wiping of a floor surface, of course, and even a high location of a wall surface and a ceiling easy.

Note that the base paper sheet described here means a state of the cleansing wet wipe 100 before the impregnation with the chemical solution.

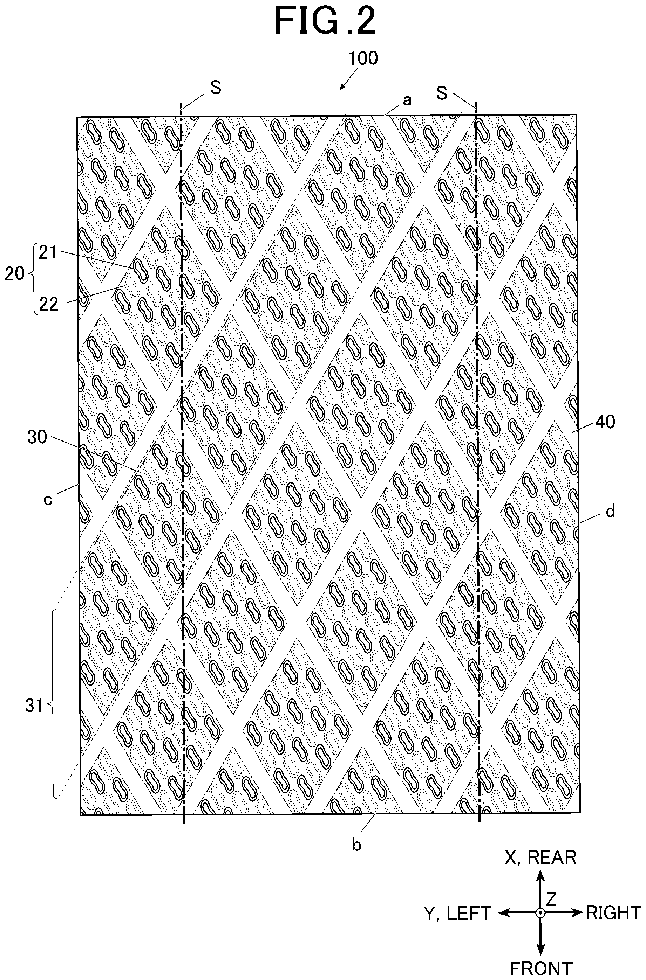

The cleansing wet wipe 100 is formed into a rectangular shape as illustrated in FIG. 2, covers a bottom surface of the head portion 201 of the cleaning tool 200 to form a cleaning surface, is folded at fold lines S along longitudinal edge portions 201a of the head portion 201 of the cleaning tool 200, is locked at an upper surface of the head portion 201, and is thereby brought into a state where the cleansing wet wipe 100 is attached to the cleaning tool 200.

Note that the longitudinal edge portions 201a denote edge portions along the longitudinal direction of the head portion 201. In other words, the longitudinal edge portions 201a denote two longer edge portions among the four edge portions of the head portion 201 with the rectangular shape.

### ((2) Base paper sheet)

The base paper sheet is a non-woven cloth manufactured by bonding predetermined fibers by a known technique such as spunlace, air-through, air-laid, point-bond, spunbond, or needle punch.

Specifically, a non-woven cloth manufactured by mixing hydrophilic fibers and hydrophobic fibers and bonding these fibers, for example, is used as the base paper sheet.

### (a. Hydrophilic fibers)

It is possible to use, as the hydrophilic fibers, natural fibers such as cotton, pulp, or hemp, recycled fibers such as rayon or cupro, or the like, and it is preferable to use pulp, rayon, polypropylene spunbond fibers (PPSB), or the like from the viewpoint of maintaining water retention properties.

Also, a blending ratio of the hydrophilic fibers in the fibers constituting the base paper sheet is preferably from 35% by mass to 75% by mass.

### (b. Hydrophobic fibers)

Examples of the hydrophobic fibers include polyolefin-based fibers such as polyethylene (PE), polypropylene (PP), and polyvinyl alcohol, polyester-based fibers such as polyethylene terephthalate (PET) and polybutylene terephthalate (PBT), acrylic fiber, and the like, and one of these can be used alone, or two or more kinds of these can be used in combination. Examples of composite fibers of two or more kinds include a core-in-sheath type including a resin (low-melting-point resin) having a relatively low melting point as a sheath portion and a resin (high-melting-point resin) having a relatively high melting point as a core portion, a sideback type in which a low-melting-point resin and a high-melting-point resin are aligned in a predetermined direction, and the like.

Also, a blending ratio of the hydrophobic fibers in the fibers constituting the base paper sheet is preferably from 25% by mass to 65% by mass.

### (c. Basis weight)

The basis weight of the base paper sheet is preferably from 50 g/m² to 100 g/m² from the viewpoint of achieving both a contamination retention ability and sheet flexibility. Note that the basis weight is a paper weight measured in accordance with JIS P8124:2011.

### ((3) Chemical solution)

As the chemical solution with which the base paper sheet as described above is impregnated, a chemical solution containing at least iodopropynyl butylcarbamate, polyaminopropyl biguanide, benzalkonium chloride, and cetylpyridinium chloride is used.

Iodopropynyl butylcarbamate (IPBC) is an antiseptic agent that shows an antiseptic effect mainly against fungi. In the chemical solution, content of iodopropynyl butylcarbamate is preferably from 0.005% by mass to 0.02% by mass. Within this range, a manufacturing cost is reduced within a proper range, and a satisfactory anti-mold effect is exhibited.

Polyaminopropyl biguanide is a cationic antiseptic agent. In the chemical solution, the content of polyaminopropyl biguanide is preferably from 0.05% by mass to 0.1% by mass. Within this range, a manufacturing cost is reduced within a proper range, and a satisfactory anti-mold effect is exhibited.

Benzalkonium chloride is a cationic antiseptic agent. In the chemical solution, the content of benzalkonium chloride is preferably from 0.05% by mass to 0.125% by mass. The antiseptic effect is unlikely to be expressed if the content is less than 0.05% by mass, and there is a concern that skin irritation increases if the content exceeds 0.125% by mass.

Cetylpyridinium chloride is a cationic surfactant having a high anti-mold effect. In the chemical solution, the content of cetylpyridinium chloride is preferably from 0.01% by mass to 0.03% by mass. The antiseptic effect is unlikely to be expressed if the content is less than 0.01% by mass, and there is a concern that foaming occurs and leads to a trouble in a manufacturing process if the content exceeds 0.03% by mass.

Also, 150% by mass to 450% by mass, preferably 180% by mass to 350% by mass of chemical agent is used for impregnation with respect to the mass of the base paper sheet when the base paper sheet is dry.

The dried base paper sheet is impregnated with the chemical solution, and the chemical solution is released from a cleaning surface when the cleansing wet wipe 100 is used.

### ((4) Embossed parts)

As illustrated in FIGS. 1 and 2, embossed parts 20 which are parts where the sheet is compressed in the Z direction are arranged in the cleansing wet wipe 100 in order to improving wiping performance against contamination on a cleaned surface by generating unevenness in the sheet and scraping off the contamination with the unevenness.

The embossed parts 20 have a thin and long oval shape with a short width in one direction and is formed into a so-called gourd shape having a narrowed portion at a substantially center in a long axis direction in a plan view as illustrated in FIG. 2, for example. The shape of the embossed parts 20 is not limited thereto, and various shapes such as polygonal shapes or shapes obtained by combining such shapes may be adopted. However, such a gourd shape is preferably adopted from the viewpoint of improving contamination scraping properties.

Such embossed parts 20 can be formed through heat embossing under the conditions of a temperature of 80°C to 200°C and an embossing pressure of 0.2 MPa to 1.0 MPa. In a case where the embossed parts 20 are formed through heat embossing, it is possible to use, as a projecting emboss roll, a projecting emboss roll with at least outer peripheral surface made of carbon steel, stainless steel, a cured resin such as polypropylene or acrylonitrile butadiene styrene (ABS) resin, or the like, and among these, it is preferable to use a projecting emboss roll made of stainless steel from the viewpoint of durability and heat resistance.

Moreover, in a case where emboss working is performed by heat embossing, it is preferable to perform the emboss working before the process of impregnating the cleansing wet wipe 100 with the chemical solution from the viewpoint of easiness in application of the uneven shape.

### (a. Projecting embossed parts, recessed embossed parts)

As the embossed parts 20, projecting embossed parts 21 that have a shape projecting upward in the Z direction (on a side of a first surface of the cleansing wet wipe 100) and recessed embossed parts 22 that have a shape projecting downward (on a side of a second surface of the cleansing wet wipe 100) (that is, a shape recessed upward in the Z-direction) are formed. Note that in each drawing, the projecting embossed parts 21 are illustrated by solid lines and the recessed embossed parts 22 are illustrated by dashed lines.

The projecting embossed parts 21 are formed to 5 mm to 10 mm, or preferably 6 mm to 8 mm in the long axis direction, are formed to 2 mm to 5 mm, or preferably 3 mm to 4 mm in a short axis direction perpendicularly intersecting the long axis direction, and are formed to 0.5 mm to 2 mm, or preferably 0.7 mm to 1.5 mm in the Z direction (the height from an intermediate portion (which will be described later)). The recessed embossed parts 22 are formed upside down with respect to the projecting embossed parts 21 and with substantially the same shape, and is formed into a shape projecting downward in the Z direction in a sectional view.

### (b. Intermediate portion)

Intermediate portions are formed between the embossed parts 20 formed in the cleansing wet wipe 100. Since the intermediate portions are parts where the embossed parts 20 are not formed, the intermediate portions are located at a position that is lower than the projecting embossed parts 21 and higher than the recessed embossed parts 22 in the Z direction.

### (c. Emboss pattern)

In the cleansing wet wipe 100 according to the present embodiment, first emboss blocks 30 with a rhomboidal shape, in each of which a combination of the projecting embossed parts 21 and the recessed embossed parts 22 is arranged such that an angle formed by a first direction (the X direction in FIG. 2) perpendicularly intersecting a first side a and the long axis direction is 5° to 45°, or preferably 15° to 35°, continue from the first side a to a second side b to form an emboss block array 31, a plurality of emboss block arrays 31 are continuously arranged from a third side c to a fourth side d, and arrangement is made such that in a first emboss block array 31 and a second emboss block array 31 that are adjacent to each other, first emboss blocks 30 in the first emboss block array 31 overlaps adjacent second emboss blocks 30 in the second emboss block 31 array in a second direction (the Y direction in FIG. 2) perpendicularly intersecting the first direction and at least one projecting embossed part 21 and at least one recessed embossed part 22 are present on a straight line extended perpendicularly to the fourth side d from an arbitrary point on the third side c.

### (d. Non-embossed portion)

As illustrated in FIG. 2, non-embossed portions 40 that are parts which are not compressed in the Z direction as compared with the parts where the embossed parts 20 are formed in the cleansing wet wipe 100 and which have raising nap are provided between the adjacent emboss blocks 30.

The non-embossed portions 40 can be formed by designing the projecting emboss roll for forming the embossed parts 20 to exclude the shape of the non-embossed portions 40, or alternatively, by slightly performing compression as compared with the compressed parts of the embossed parts 20 to an extent to which the raising nap remains.

It is possible to obtain the cleansing wet wipe 100 that reduces occurrence of wiping streaks and has excellent wiping properties by such non-embossed portions 40 being provided at a proportion of 25% to 50% with respect to the area of the cleansing wet wipe 100. Note that it is most preferable that the non-embossed portions 40 be provided at a proportion of 25% with respect to the area of the cleansing wet wipe 100.

Note that although the cleaning tool 200 is typically moved substantially perpendicularly to the X direction or the Y direction in a case where wiping is performed with the cleaning tool 200 with the cleansing wet wipe 100 attached thereto, there is a concern that in a case where the non-embossed portions 40 are formed in a straight line shape as illustrated in FIG. 2, wiping streaks may be likely to occur depending on an angle at which the cleansing wet wipe 100 is moved. Thus, it is more desirable that the emboss blocks 30 be arranged such that the non-embossed portions 40 are not on straight lines.

### (2. Description of effects)

The cleansing wet wipe 100 according to the present embodiment is provided with the emboss blocks 30 (emboss block arrays 31), in each of which the plurality of projecting embossed parts 21 that are embossed parts projecting from the first surface and the plurality of recessed embossed parts 22 that are embossed parts projecting from the second surface are formed.

Such a cleansing wet wipe 100 enables wiping of scraping off contamination on a cleaned surface with unevenness of the embossed parts 20 (projecting embossed parts 21, recessed embossed parts 22) and suitably wiping off the contamination.

In particular, the cleansing wet wipe 100 according to the present embodiment is impregnated with the chemical solution that contains iodopropynyl butylcarbamate, polyaminopropyl biguanide, benzalkonium chloride, and cetylpyridinium chloride and has anti-mold performance, and it is thus possible to apply the chemical solution to a cleaned surface wiped with the cleansing wet wipe 100 and to apply an anti-mold effect to the cleaned surface.

In this manner, the cleansing wet wipe 100 according to the present embodiment can apply the anti-mold effect to the cleaned surface after wiping, and it is thus possible to apply the anti-mold effect to the cleaned surface while wiping off mold contamination caused in a bathroom, for example.

Also, if the cleansing wet wipe 100 is attached to the head portion 201 of the cleaning tool 200 including the handle portion 202 with the long bar shape and is used therewith as illustrated in FIG. 1, it is possible to suitably wipe a high location such as a ceiling of a bathroom and to thereby more easily perform mold cleaning of the bathroom than by spraying a chemical solution and performing cleaning.

Also, according to the cleansing wet wipe 100 of the present embodiment, the embossed parts 20 are arranged as described above, and it is thus possible to improve wiping performance against contamination by the unevenness of the sheet and to obtain the following effects.

In other words, when the cleansing wet wipe 100 is attached to the cleaning tool 200 including the head portion 201 and wiping is performed, the cleaning tool 200 is typically moved in the first direction (the X direction in FIG. 2) or the second direction (the Y direction in FIG. 2). However, if the projecting embossed parts are arranged in series such that the projecting embossed parts are aligned in such a first direction or second direction, locations which are not wiped by the projecting embossed parts (so-called "wiping streaks") may be caused in the cleaned surface, and a user who has visually recognized the wiping streaks may determine that the chemical solution has been used up and discard the sheets regardless of the chemical solution still remaining in the sheets, and unnecessarily use a lot of sheets.

On the contrary, in a case where the cleansing wet wipe 100 which has the emboss pattern as described above and includes at least one projecting embossed part 21 arranged to be present on the straight line extended perpendicularly to the fourth side d from an arbitrary point on the third side c is attached, the projecting embossed parts 21 have overlapping margins, and it is thus possible to reduce occurrence of wiping streaks when wiping is performed at least in the second direction (the Y direction in FIG. 2).

In addition, the projecting embossed parts 21 receive larger pressures than the non-embossed portions 40 when wiping is performed with the cleansing wet wipe 100 by providing the non-embossed portions 40 at a proportion of 25% to 50% with respect to the area of the cleansing wet wipe 100 between the emboss blocks 30 without providing the embossed parts 20 over the entire surface of the cleansing wet wipe 100, chemical solution releasing properties are thus improved, and it is possible to further reduce occurrence of wiping streaks.

Also, since scraping properties of the projecting embossed parts 21 are improved and dirt is likely to be accumulated in the non-embossed portions 40 by the projecting embossed parts 21 receiving large pressures, it is possible to enhance dirt capturing properties. Moreover, regardless of the enhancement of the scraping properties of the projecting embossed parts 21 and the dirt capturing properties of the cleansing wet wipe 100, it is possible to suppress an increase in wiping resistance because the non-embossed portions 40 are unlikely to be grounded on the cleaned surface due to the projecting embossed parts 21.

In addition, since the emboss blocks 30 have a rhomboidal shape and include embossed parts 20 arranged such that the angle formed by the first direction and the long axis direction is 5° to 45°, or preferably 15° to 35°, the emboss blocks 30 are inevitably arranged with deviation in the first direction of the cleansing wet wipe 100 when the plurality of emboss block arrays 31 continuously arranged from the first side a to the second side b are continuously arranged from the third side c to the fourth side d. Also, a margin occurs in the angle at which the user moves the cleansing wet wipe 100 when the user performs wiping.

Furthermore, since the embossed parts 20 have an oval shape and have a shape with a narrowed portion at substantially the center in the long axis direction, it is possible to enhance contamination scraping performance.

### Examples

Next, results of evaluating examples of the present embodiment will be described. Although the present invention will be specifically described below on the basis of the examples, the present invention is not limited thereto.

### (Production of chemical solution samples)

As chemical solutions for mold cleaning, a chemical solution of an acidic formulation (pH 3.16) and a chemical solution of an alkaline formulation (pH 8.91) were prepared using constituents and blending ratios (% by mass) shown in Table 1.

**[Table 1]**

| | Acidic formulation[%] | Alkaline formulation [%] |
|---|---|---|
| Distilled water | 88.94 | 88.43 |
| Ethanol | 10 | 10 |
| Phenoxyethanol | 0.2 | 0.2 |
| Polyoxyethylene alkyl ether | 0.1 | 0.1 |
| Grapefruit seed extract | 0.05 | 0.05 |
| Cetylpyridinium chloride | 0.03 | 0.03 |
| 50% aqueous solution of benzalkonium chloride | 0.1 | 0.1 |
| Polyaminopropyl biguanide (20% product) | 0.5 | 0.5 |
| Iodopropynyl butylcarbamate (IPBC) | 0.02 | 0.02 |
| Citric acid | 0.05 | - |
| Sodium citrate | 0.01 | - |
| 5% aqueous solution of sodium hydroxide | - | 0.3 |
| Glycine | - | 0.15 |
| Sodium chloride | - | 0.12 |
| pH | 3.16 | 8.91 |

Also, as a base paper sheet, a base paper sheet obtained by performing emboss working illustrated in FIG. 2 on a non-woven cloth that had a fiber blending ratio of PET/pulp/PP/PP·PE = 45/35/15/5 and a basis weight of 100 g/m² was used.

A sample of a cleansing wet wipe obtained by impregnating the base paper sheet with 200% of chemical solution of the acidic formulation and a sample of a cleansing wet wipe obtained by impregnating the base paper sheet with 200% of chemical solution of the alkaline formulation were produced.

### (Anti-mold test method)

A test simulating wiping of a surface of a fiber-reinforced plastic panel (FRP panel), which was often used in a unit bath, with the cleansing wet wipe 100 was conducted.

Here, five FRP panels with the size of 50 mm × 50 mm were aligned and set up in the left-right direction, and a sample of the cleansing wet wipe was placed at the right end thereof, and a weight of 150 g was put on the sample.

Then, the sample of the cleansing wet wipe in the state where the weight was put thereon was moved to the left end of the five FRP panels in about two seconds, and was then moved from the left end to the right end in about two seconds.

In this manner, mock wiping of causing the sample of the cleansing wet wipe placed on the FRP panels to reciprocate once was conducted. Specifically, the sample of the cleansing wet wipe was fixed to a bottom of a 300 ml beaker, about 10 ml of water was put into the beaker, and the thus obtained object with the weight of 150 g was caused to reciprocate once. Note that the FRP panel set up at the center from among the five FRP panels was used for the test.

In this manner, two FRP panels wiped with the sample of the cleansing wet wipe impregnated with the chemical solution of the acidic formulation and two FRP panels wiped with the sample of the cleansing wet wipe impregnated with the chemical solution of the alkaline formulation were obtained.

Note that 150 g of the weight was set to simulate wiping using the cleaning tool 200 as a floor wiper.

One of the FRP panels wiped with each sample was naturally dried after the mock wiping.

In this manner, an acidic formulation wiped specimen (specimen A) and an alkaline formulation wiped specimen (specimen B) were obtained.

Also, 5 ml of distilled water was sprayed onto the cleaned surface of the remaining one of the FRP panels wiped with each sample using a mist spray, and the FRP panel was then naturally dried.

In this manner, a specimen that was subjected to rinsing with water after the acidic formulation wiping (specimen C) and a specimen that was subjected to rinsing with water after the alkaline formulation wiping (specimen D) were obtained.

Note that 5 ml of the distilled water was set to simulate the amount of water when water was poured to 5 cm² of cleaned surface with shower for 0.5 seconds.

Next, each of the above four types of specimens (A to D) was put on a glucose-added inorganic salt agar plate medium inside a petri dish, the entire surfaces of each specimen and the agar plate medium were inoculated with 0.1 mL of mixed spore suspension, and the agar plate medium was cultivated for four weeks inside a constant-temperature reservoir (in an environment at 29 ± 1°C and with a relative humidity of equal to or greater than 95%).

Mold that had grown inside the petri dish was observed, and mold growth states were evaluated in accordance with the following six levels.

Note that the anti-mold test was a test following JIS Z 2911, and if there was a difference corresponding to one or more levels in comparison with an untreated specimen, the effect was recognized.

### Six-level evaluation

"0": No growth of mold was recognized with naked eyes and with a microscope.
"1": Although growth of mold was not recognized with naked eyes, the grow was clearly observed with a microscope.
"2": Growth of mold was recognized with naked eyes, and the area of the growth part was less than 25% of the entire area of the specimen.
"3": Growth of mold was recognized with naked eyes, and the area of the growth part was equal to or greater than 25% and less than 50% of the entire area of the specimen.
"4": Hyphae grew well, and the area of the growth part was equal to or greater than 50% of the entire area of the specimen.
"5": Hyphae grew vigorously and covered the entire surface of the specimen.

### (Test Results)

Evaluation of the four types of specimens (A to D) and a blank will be shown below.
- Acidic formulation wiped specimen (specimen A); evaluation "3"
- Alkaline formulation wiped specimen (specimen B); evaluation "1"
- Acidic formulation-water rinsed specimen (specimen C); evaluation "4"
- Alkaline formulation-water rinsed specimen (specimen D); evaluation "4"
- Untreated specimen (blank); evaluation "5"

It is possible to ascertain from the results that anti-mold effect was applied to the four types of specimens (A to D) .

In particular, it is possible to ascertain that the anti-mold effect applied to the specimen B that was subjected to mock wiping with the sheet that was impregnated with the chemical solution of the alkaline formulation was high.

It is possible to ascertain that a sufficient anti-mold effect was also applied to the specimen A that was subjected to mock wiping with the sheet that was impregnated with the chemical solution of the acidic formulation.

Moreover, it is possible to ascertain that the anti-mold effect was degraded if water was poured to the cleaned surface with shower after wiping with the cleansing wet wipe.

According to the above results, both the cleansing wet wipe 100 that is impregnated with the chemical solution of the alkaline formulation and the cleansing wet wipe 100 that is impregnated with the chemical solution of the acidic formulation can apply the anti-mold effect to the cleaned surface after wiping.

In particular, the anti-mold effect of the cleansing wet wipe 100 that is impregnated with the chemical solution of the alkaline formulation (the chemical solution that shows alkalinity) is high.

Also, if the cleansing wet wipe 100 is provided with the emboss blocks 30 (emboss block arrays 31) as illustrated in FIG. 2, it is possible to scrape off contamination on the cleaned surface with the unevenness of the embossed parts 20 (the projecting embossed parts 21, the recessed embossed parts 22) with both the alkaline and acidic chemical solutions and to perform wiping of suitably wiping off the contamination.

For cleaning in bathrooms to remove traces of water stain or soap scum, which is so-called "scale stain", in particular, the cleansing wet wipe 100 that is impregnated with the chemical solution of the acidic formulation exhibits a higher contamination removal rate and can lead to more satisfactory cleaning.

In other words, the cleansing wet wipe 100 that is impregnated with the chemical solution of the acidic formulation (the chemical solution that shows acidity) can lead to satisfactory cleaning of wiping off contamination as a wet wipe for bathroom cleaning and suppress mold caused on the cleaned surface after the wiping.

As described above, according to the cleansing wet wipe 100 of the present embodiment, it is possible to suitably wipe off contamination and to suppress mold caused on the cleaned surface after the wiping.

Also, if the cleansing wet wipe 100 is attached to the head portion 201 of the cleaning tool 200 (floor wiper) including the handle portion 202 with the long bar shape and is used therewith, it is possible to suitably wipe a high location such as a ceiling of a bathroom and the like and to thereby more easily perform mold cleaning of the bathroom than cleaning it by spraying a chemical solution or cleaning it by bringing a stepladder or the like to the bathroom.

In other words, if the cleansing wet wipe 100 of the present embodiment and the cleaning tool 200 such as a floor wipe are used in combination to wipe a floor surface and a wall surface, of course, and also a high location such as a ceiling for which it is difficult to perform mold cleaning, mold caused on the cleaned surface is suppressed, and the bathroom can be easily maintained in a clean state, without using any anti-mold smoking agent.

Note that although the case where the bathroom is cleaned using the cleansing wet wipe 100 has been described as an example in the above embodiment, the present invention is not limited thereto, the cleansing wet wipe 100 may be used to clean basin areas such as a washroom and a toilet, and wiping may be performed by holding the cleansing wet wipe 100 in a hand without using the cleaning tool 200.

It is a matter of course that other specific detailed structures and the like can also be appropriately modified.

### Industrial Applicability

The present invention is configured as described above and can thus be used as a cleansing wet wipe.

### Reference Signs List

- 100: cleansing wet wipe
- 20: embossed part
- 21: projecting embossed part
- 22: recessed embossed part
- 30: emboss block
- 31: emboss block array
- 40: non-embossed portion
- 200: cleaning tool
- 201: head portion
- 202: handle portion
- a: first side
- b: second side
- c: third side
- d: fourth side

## Claims

1. A cleansing wet wipe obtained by impregnating a base paper sheet with a chemical solution,
wherein the chemical solution contains at least iodopropynyl butylcarbamate, polyaminopropyl biguanide, benzalkonium chloride, and cetylpyridinium chloride.

2. The cleansing wet wipe according to claim 1, wherein in the chemical solution, content of iodopropynyl butylcarbamate is equal to or greater than 0.005% by mass and equal to or less than 0.02% by mass.

3. The cleansing wet wipe according to claim 1, wherein the chemical solution is adjusted to show acidity.

4. The cleansing wet wipe according to any one of claims 1 to 3, wherein a plurality of projecting embossed parts which are embossed parts projecting from a first surface and a plurality of recessed embossed parts which are embossed parts projecting from a second surface are formed in the base paper sheet.

5. The cleansing wet wipe according to claim 4, wherein the cleansing wet wipe is a wet wipe for cleaning a bathroom.
